# EUROPEAN PATENT SPECIFICATION

(11) **EP 0 673 352 B1**
(45) Date of publication and mention of the grant of the patent: **21.04.1999**
(21) Application number: 94901971.5
(22) Date of filing: 10.12.1993
(51) Int. Cl.: C07C 2/12, C07C 2/58

(54) **PROCESS AND REACTOR SYSTEM FOR OLIGOMERIZATION OF OLEFINS**
VERFAHREN UND ANLAGE ZUR OLIGOMERISIERUNG VON OLEFINEN
PROCEDE ET SYSTEME REACTIONNEL POUR L'OLIGOMERISATION DES OLEFINES

(30) Priority: 10.12.1992 FI 925608
(43) Date of publication of application: 27.09.1995
(73) Proprietor: Neste Oyj, 02150 Espoo (FI)
(72) Inventor: KESKINEN, Kari, I., FIN-01380 Vantaa (FI)
(74) Representative: Bannerman, David Gardner
(86) International application number: FI9300535
(87) International publication number: WO9413599

(56) References cited:
- EP-A- 0 397 273
- US-A- 4 935 577
- US-A- 4 950 834

## Description

The present invention concerns a process according to the preamble of claim 1 for oligomerization of olefins.

According to the present process, a feedstock containing C₃ - C₂₀ olefins, or a mixture thereof, is fed to a distillation reactor system, where said olefins of the feedstock are contacted with a catalyst in order to produce an oligomer-containing product.

The invention also concerns a reactor system according to the preamble of claim 25 for oligomerization of olefins.

Oligomerization of olefins aims at converting light olefins into heavier olefins. This reaction is carried out in the presence of a catalyst, which comprises either an acid or a base. Conventionally, a heterogeneous catalyst is used. Thus, a mixture of light olefins in the liquid phase, gas phase or a mixture thereof, or in supercritical phase are usually contacted with a solid phase catalyst. Then, the olefins combine with each other forming dimers, trimers, tetramers and even heavier oligomers. The degree of oligomerization depends on the pressure and temperature employed.

If the reaction temperature used is high (above 200 °C), depending on the catalyst type, cracking products are produced by side reactions, and disproportionation reactions will give rise to other products different from the oligomers contained in the olefin stream used as feedstock. Thus, the properties of the catalyst employed have an extremely strong influence on the composition of the products obtained.

Heavier olefins from the oligomerization processes can be utilized as, for instance, gasoline and diesel fuel blending stock, solvents and lubricant stock, all of which can further be hydrogenated to improve their stability, or alternatively, as feedstocks for various chemical processes using heavy olefins. Also in the latter case, the mixture of the olefins must be separated by, e.g., distillation into proper fractions. Thus, the product being manufactured via oligomerization can be controlled by an appropriate choice of reaction conditions, catalyst used and the mixture of olefins employed as feedstock.

In many of the above-listed applications, a benefit of the products manufactured by oligomerization is their freedom from sulfur (namely, sulfur compounds can be removed to a high accuracy from light hydrocarbons) and low content of aromatics, in many cases far below one percent.

Processes intended for oligomerization of light olefins have been developed by, e.g., Mobil (MOGD process), Shell (SPGK process), IFP (Dimersol, Selectopol and Polynaphtha processes), UOP (Hexall process); catalytic polymerization (using phosphoric acid as a liquid-phase catalyst or impregnated on a carrier); and further, by Bayer, Hüls/UOP (Octol process).

Catalytic polymerization mentioned above, which uses a liquid catalyst, is complicated in that waste acid is produced. By contrast, most of the other processes listed by reference employ a solid phase catalyst, and they are run in a tubular reactor or a configuration of multiple tubular reactors. The reason for the use of multiple-reactor configurations lies in the exothermic nature of the oligomerization reaction. To control the temperature in a system of multiple reactors, intermediate cooling of the reaction mixture can be arranged between the reactors. Another frequently employed arrangement is to recycle the product back to the reactor feed after passing a possible distillation unit or flash drum unit. The latter system produces heavier olefins and simultaneously achieves temperature control for the reactor, because the oligomerization of heavier olefins releases less heat per mass unit than the oligomerization of lighter olefins. A third alternative employed for solving the energy balance problems is the recycling of already processed, inert paraffinic components in order to control the temperature in the reactors.

An example of embodiments based on intermediate cooling and reflux of inert paraffins is provided by the process disclosed in EP Patent Application No. 0,397,273. According to said publication, a hydrocarbon flow containing light olefins is taken via heat exchangers and a boiler to two series-connected reactors in which the olefins are contacted with a zeolite catalyst. The hydrocarbon flow is subjected to intermediate cooling between the reactors and the product flow from the second reactor is subjected to additional cooling prior to further processing. During further processing, the butane-containing fraction is separated from the product in a distillation column. The oligomerized hydrocarbons are removed as the bottom product from the distillation column. The distillate from the distillation column is condensed and a portion thereof is recycled back to the column, while the remaining portion containing mostly C₃ and C₄ alkanes is recycled to the reactors.

A specific problem of processes based on intermediate cooling is the inefficient recovery of reaction heat, which results in a greater number of equipment required. The feedstock and product flows in these processes must be alternatingly first heated and then cooled. Moreover, heat produced separately must be introduced to the fractionation step of the products. Neither is the recycling of inert paraffins an ideal solution in terms of process energy utilization.

It is an object of the present invention to overcome the drawbacks of the above-described conventional techniques and to achieve an entirely novel process and reactor system for the oligomerization of olefins.

The invention is based on the basic concept of carrying out the oligomerization reaction and the fractionation of the inert paraffins from the product in a single system of apparatuses and utilizing the released reaction heat in said fractionation step. In short, the present embodiment is based on catalytic distillation and modifications thereof.

The term "catalytic distillation" (also known as "reactive distillation") conventionally refers to the combination of a chemical reaction with the fractionation of products. In distillation the reaction of feedstock reactants and the fractionation of the product components take place simultaneously. A catalytic distillation apparatus typically comprises a distillation column including one or more catalyst zones. In a catalyst zone a product obtained from a certain level or tray of the distillation column is processed to form desired reaction product components, after which the product stream thus obtained is fractionated. Catalytic distillation is employed in, e.g., the manufacture of tertiary ethers, where it offers significant benefits in terms of reaction kinetics.

In the prior art, catalytic distillation has also been used in oligomerization processes. Thus, US Patent Specification No. 4,935,577 anticipates an oligomerization process in which alpha-olefins containing 3 to 12 carbon atoms are fed to a distillation column, where they are reacted with a catalyst which comprises a Lewis acid and a non-zeolitic inorganic oxide. The catalytic distillation unit is maintained at a maximum temperature of approx. 150 °C, preferably below approx. 50 °C. As the process uses a combination catalyst, the equipment must incorporate a specific unit for separating the Lewis acid, typically BF₃, from the propane flow for recycling back to the distillation unit.

US Patent Specification No. 2,486,533 describes a process for polymerization of olefinic hydrocarbon monomers in the presence of a solid phosphoric acid catalyst, water vapor being used to maintain the catalyst coposite at the desired degree of hydration. The process is implemented in an apparatus comprising a centrally located polymerization reactor, a monomer stripping section positioned above the reactor and a polymer stripping section situated below said reactor. According to an alternative embodiment, the reactor and the monomer and polymer stripping sections can be combined to form a single continuous unit of apparatus maintained at a uniform pressure throughout.

The present invention differs from the above-described prior-art techniques in several respects. The present invention utilizes a solid phase catalyst suited for oligomerization of olefins, i.e. a "solid oligomerization catalyst". Only such solid catalysts are employed which will function without any separate adjuvants, such as water (US 2,486,533) or BF₃ (US 4,935,577), which have to be separately removed from the catalytic system. Said catalyst can be placed directly in the distillation apparatus. However, the present invention particularly concerns a process and an reactor system in which the solid catalyst is placed in a separate reactor connected to said distillation column in a manner that permits recycling of the sidestream flow obtained from the column back to the column after being subject to a conversion reaction in said reactor. The olefins are contacted with the catalyst at a temperature sufficiently high to attain oligomerization, and at least a portion of the product flow leaving the catalyst zone is fractionated in the same distillation column.

A further difference to the prior art is that it is an object of the present invention to oligomerize all olefins, not only alpha-olefins.

More specifically, the process in accordance with the invention is principally characterized by what is stated in the characterizing part of claim 1.

In the context of the present application the term "catalytic distillation" is used for denoting a combination of a reaction with the fractionation of products by distillation so that
- an essential portion, preferably at least 20 %, of the internal liquid flow through the distillation column is routed through the catalyst zone and
- the portion of liquid flow routed through the catalyst zone is returned to the distillation column at a point which is most desirable for the distillation process.

The above-mentioned characteristics are important to the end of achieving the goals of the invention. Consequently, such an arrangement in which a relatively small sidestream flow is taken from the distillation column intended for the fractionation of the products of the oligomerization process and routed to prereactors fails to utilize the heat released by the exothermic reaction in the improvement of the energy balance of the distillation process. Accordingly, such an alternative does not represent "catalytic distillation" in the meaning of the present application.

It should further be noticed that the side reactor according to the present invention preferably comprises a flow reactor, such as a tubular reactor or equivalent, which is operated in such a way that no fractionation of the products will take place therein; the reactants and the reaction products flow in the same direction through the side reactor. Although there usually is only one inlet in the side reactor, i.e. the one for the feed drawn off from the distillation column, it is in principle possible to feed two or more streams into the side reactor by using one or several additional inlets. All the reaction product is, however, withdrawn from the side reactor from one point and the fractionation of the product is carried out in a distillation column or in a reactive distillation column.

In the following description, the terms "catalyst zone" and "catalyst layer" will be used interchangeably.

The catalysts suited for use according to the invention can be, e.g., zeolite catalysts or their ion-exchanged variations. Typical zeolites incorporate crystalline aluminosilicate zeolites such as, e.g., ZSM-5 zeolite presented in US patent publication 3,702,886 and variants thereof such as ZSM-11, ZSM-12, ZSM-23, ZSM-35, and ZSM-38 for instance. Other suitable catalysts are, e.g., borosilicates, ferrosilicates and/or aluminosilicates. As catalysts, HSiW and other hetero polyacid -type catalysts can also be used. However, the invention is not limited to the above-named catalyst grades alone, but rather, all such solid phase catalysts that catalyze the oligomerization reactions of olefins are suited for use according to the invention.

The catalyst is preferably a zeolite, and the reaction is carried out at a minimum temperature of 150 °C.

If several catalyst layers are used in embodiments of the invention, be it inside the distillation column, in the side reactors or in both the distillation column and the side reactors, the various catalyst layers may contain catalysts of different types. This makes it possible to use for each respective location of the layer the catalyst type that works best. As a result, some heating and cooling phases may become superfluous and can therefore be avoided.

As an additional benefit of using different types of catalysts in the same reaction system, it may be pointed out that, working in different temperature ranges, different catalyst have different selectivity and will provide products with different product distributions and various amounts of sideproducts. It is therefore possible to modify the properties of the products by the selection of the catalyst types.

The use of different catalysts can be illustrated by a process in which the upper side reactor of a conventional distillation column contains ZSM-5 zeolite, which will catalyze oligomerization of light olefins, and the lower side reactor of said column contains a Ni modified zeolite which only catalyzes dimerization. This embodiment makes it possible to change both the ratio between the diesel oil and lubricant oil fractions and the quality of said fractions.

Another example of the use of different catalysts is indicated in Example 5 below, wherein a ZSM-5 zeolite and a HSiW (hetero polyacid) -type catalyst are employed.

The catalyst for the reaction is placed in either the distillation apparatus, or alternatively, in at least one separate reactor (later called the "side reactor") connected to said distillation column, the sidestream flow from the distillation column to said side reactor(s) being returned from the reaction zone of said side reactor(s) back to the column after being converted in the reaction.

An embodiment according to the invention incorporates at least one catalyst layer which is arranged in conjunction with the trays located above the feed tray of the distillation column, a reaction consuming light olefins being achieved.

The invention uses a "solid", contiguous catalyst, thus avoiding the need for any auxiliary equipment for separating the catalyst from the products.. Consequently, the process can be fed with such feedstock mixtures that contain inert components in relatively high concentrations. Therefore, the process feedstock can comprise a mixture of olefins and paraffins of approximately equal boiling point ranges. The olefins will be oligomerized and removed as heavier fractions from the column bottom, while the paraffins as inert compounds leave the column from its top. The lower limit of the olefinic components concentration is approx. 10 %, while preferably the feedstock contains at least approx. 50 % of olefins. The feedstock can then, e.g., include the unreacted part of the feedstock (also known as Raffinate) with a high content of alkanes that is available in the product stream of a preceding oil refining unit. Accordingly, the process can be fed with a mixture containing 40 % butane and approx. 60 % butenes. It is also possible to feed the process with mixtures containing, in addition to C₄ hydrocarbons, also C₃ hydrocarbons (cf. Example 5). In fact, as Example 6 below shows, the feed may even primarily be comprised of C₃ hydrocarbons. Thus, a propene feedstock containing approx. 20 % propane as an impurity can be oligomerized in the presence of ZSM-5 type catalysts to produce lubricant fractions having high viscosity indeces.

The paraffins of the feedstock are inert and provide temperature stabilization for such a reaction in which mixtures with a high content of light olefins are oligomerized. The feedstock can also contain heavy paraffins such as C₁₀₊ that are directly suitable for the product composition without hydrogenation.

The basic concept of the invention can be implemented by carrying out an oligomerization process in a conventional catalytic distillation column with the catalyst packed in the column. As a difference to the prior art, according to a specific embodiment of the basic concept, the solid catalyst used then at least substantially consists of a zeolite, the olefins are contacted with the catalyst at a temperature sufficiently high for oligomerization, and at least a portion of the product flow leaving the catalyst zone is fractionated in the same distillation column. A prereactor can be connected to the reactor for increasing the proportion of heavy olefins in the feed flow of the initial reactants. Correspondingly, a postreactor can be connected after the column for further conversion of the product stream from the column into yet heavier olefins. The catalyst is arranged in at least one, preferably in 2 to 5 reaction zones having, when necessary, heater elements adapted within the zones such as tubular heaters for instance, suited for heating the reaction zones independently from each other. Thus, for instance, with ZSM-5 type zeolite packed in reaction zones, the temperatures of the various zones are maintained the higher the lower the zone is located in the column. Typically, the temperatures of the reaction zones are in the range from 150 to 350 °C, preferably from 150 to 320 °C and in particular approx. 180 to 300 °C. However, operation at a lower temperature is also possible provided that the catalyst employed can perform satisfactorily at such temperatures. In exemplifying embodiments 1 and 2 discussed later, a distillation column is described having three reaction zones in which the reaction zone temperatures are (from top to bottom): 180 to 220 °C, 255 to 260 °C and 265 to 280 °C.

The distillation column can be of any conventional type such as a tray column with sieve, bubble cap or valve trays or a packed filling column. The term "tray" (e.g. the "feed tray") herein refers to either a physical or a theoretical tray.

Instead of a single column, two or more columns can be used in the process, each of which performs the oligomerization of olefins. In this case the columns are preferably connected in series. Each column can have one or more reaction zones in the manner described above.

According to a first preferred embodiment of the invention, the oligomerization reactions are performed at least partially in side reactors. The process feed can be routed partially or entirely to the side reactors, whereby the distillation column does not necessarily have a direct feed. This condition also applies to that alternative embodiment according to the invention in which the olefin mixture is first routed via a prereactor for, e.g., increasing the proportion of heavy olefins, or alternatively, binding catalyst poisons, because the conversion of olefins in a configuration comprising a distillation column and a side reactor may be relatively low in terms of the ultimate use of the product. This problem can be solved particularly advantageously by arranging a separate olefin feed nozzle to at least one of the side reactors. When the initial reactant is fed to the side reactor during system startup, no concurrent sidestream feed from the distillation column is taken. By feeding the initial reactant directly to the side reactor, the product composition can also be controlled.

In a second preferred embodiment of the present invention, the reactor system of catalytic distillation comprising a distillation column and at least one side reactor is brought as close as possible to the conditions of conventional reactive distillation. The number of required reactors is dependent on the olefin content of the reacting mixture and the use of a possible prereactor. Typically, the invention is implemented using multiple side reactors to which the reflux flows from one tray of the column to another are routed, whereby the return tray is selected so as to attain maximum degree of fractionation. In an ideal case the system according to the invention approximates very closely the conditions of conventional reactive distillation.

According to the invention the minimum number of sidestream flows/reactors is one, while the maximum number thereof can be as high as the number of trays in the distillation column, depending on the desird conversion or economical aspects. The maximum number is preferably determined by the number of side reactors required for obtaining optimum distillation conditions. Preferably, an embodiment according to the invention has 1 to 20, in particular about 2 to 10 side reactor units.

In exemplifying embodiment 3 discussed later, a distillation column/reactor system is described having the catalyst placed entirely in the side reactor.

The side reactor unit may comprise only one reactor, or alternatively, it can be configured from multiple smaller reactors connected in series, parallel, or both series and parallel.

According to a preferred alternative embodiment of the invention, the catalyst charge required in the distillation column/reactor system is entirely placed in the side reactors, whereby significant benefits are obtained in terms of system maintainability, for instance.

The scope of the invention also covers systems in which at least one catalyst zone is placed in the distillation column in a conventional manner.

The sidestream flow taken from the column to the side reactor can be equal to the internal liquid reflux flow of the column (total drawoff) or smaller, however, at least 20 % of the total flow. The total drawoff scheme can be applied in cases where the column is connected to multiple reactors, whereby the sidestream flow is returned to a tray below the drawoff tray. In the latter case - which is applicable in a singe-reactor configuration - the drawoff flow is approx. 40 to 90 %, typically approx. 60 to 70 %, of the reflux flow, thus making possible its return to a suitable tray below the drawoff tray. In this case the choice of a suitable tray is determined by the conventional design rules for the optimum feed tray of a distillation column.

The sidestream flow from the column can be routed via the side reactor either from bottom to top, or alternatively, from top to bottom. For a tubular reactor and a fixed-bed reactor, both flow directions are possible, while a fluidized-bed reactor requires a flow upward from the reactor bottom.

The sidestream circulation is implemented either as forced circulation by means of a pump or as natural circulation by the thermosiphon effect, whereby the reaction heat boils the liquid causing said effect.

If pumped circulation of the hydrocarbon mixture is employed, the reactor can be a fixed-bed reactor, a tubular reactor or a fluidized-bed reactor, or any combination thereof, or a configuration of multiple reactors in series. In a system based on the thermosiphon effect, only a fixed-bed or tubular reactor is suitable, and further, of these the latter represents here an inferior alternative as the gas imparting the recirculation is condensed therein.

The volume of the catalyst charge in the side reactor can be dimensioned for 0.01 to 100 times the liquid hourly space velocity which means the volume rate of liquid throughput per hour through the reactor. A typical value is approx. 0.1 to 10 times the space volume. Expressed in engineering units, the reactor LHSV is 0.01 to 100 m³liquid/m³cat./h, a typical value being 0.1 to 10 m³liquid/m³cat./h.

If the reactor is of the fixed-bed type, the fluid superficial velocity in the reactor is dimensioned according to the catalyst manufacturer specifications, typically being in the range 5 to 30 m/h for the liquid flow. In a fluidized-bed reactor the fluid superficial velocity is 1 to 10 times the minimum fluidization velocity, typically 2 to 6 times the minimum fluidization velocity.

The operating pressure of the reactors is determined by the column operating pressure and the reactor type. When a side reactor is used in connection with heat exchangers, a pump and a back-pressure regulator, the operating conditions of the side reactor can be different from those of the distillation column. Thus, the side reactors can be operated at pressures above the distillation column pressure, a condition which is frequently advantageous in terms of reaction yield. The distillation column pressure is then, e.g., 1 to 10 bar, while the side reactor pressure is in the range from 10 to 100 bar. The operating pressure of the side reactors can also be in the same order of range as the distillation column pressure (in practice, equal to column pressure plus the pressure loss in the piping). Then, the operating temperature cf the reactors is determined by the boiling point of the hydrocarbon mixture, thus preventing the occurrence cf overheated zones in the reactor.

In a circulation based on the thermosiphon action, a part of the liquid flow through the side reactor is vaporized. Then, the side reactor can be run without the need for the pump, back-pressure regulator and heat exchangers. This is possible by virtue of the energy released in the exothermic reaction that provides a portion cf the vaporizing heat. Vaporization can occur in the side reactor, whereby the preheater is arranged prior to the side reactor, or alternatively, in a boiler arranged after the reactor. The latter approach is preferred.

When the number of the side reactor units in the oligomerization process is greater than one, the distillation column/reactor configuration can be complemented by at least one heat exchanger connected so that the outlet flow of one side reactor is used for heating the feed flow of another side reactor. Such a heat exchanger can be formed by, e.g., tubes placed inside the catalyst bed of the side reactor.

The distillation column side drawoffs can be used for achieving different olefin and inert component flows. According to a preferred embodiment of the invention, the oligomer-rich product is withdrawn from the above-described columns from a tray, which is located below the feed tray and which is selected'according to the desired composition of the product. A drawoff can also be used for withdrawal of paraffinic component streams containing, e.g., C₄ alkanes.

A system according to the invention can be connected to product fractionation and hydrogenation equipment, whereby the invention becomes suited for manufacture of intermediate distillates and lubricant stock. Example 4 below includes the analysis values of a lubricant stock distillate manufactured according to the invention.

The invention provides significant benefits. Thus, the process concept can combine the reaction step with the distillation step in a single system. This approach offers an improvement of the process energy balance. A significant benefit of the reactor system described in the present patent application is the utilization of the relatively high reaction heat from the oligomerization of olefins in the distillation step. The product obtained from the oligomerization reaction that is a mixture of hydrocarbons with different carbon chain lengths (plus abundant isomers) must always be separated into suitable fractions by, e.g., distillation. The process concept according to the present invention makes it possible to simultaneously perform out the reaction and separate the desired product from the nonreacting inert hydrocarbons (paraffins) possibly carried over from the feed.

As mentioned above, the side reactors and the distillation columns can be run under different conditions. In other words, the optimal operational ranges can be chosen independently for the reactors and the column, respectively. This makes it possible for instance to improve the activity of an ageing catalyst by increasing the temperature without there being any need for a change cf the operational temperature of the column.

An additional benefit of the process is its flexibility in product grade changes. Further notice must be made on the characterizing feature that the present reactor system permits control of product composition and qualities by virtue of column pressure, temperature and degree of fractionation.

The thermal stability properties of the lubricant stock obtained after the fractionation and hydrogenation steps are excellent.

In the following the invention is examined closer with the help of a detailed description and application examples. Alternative embodiments of the invention are illustrated in the annexed drawings in which
Fig. 1 shows the simplified process diagram of a system in which alternative the catalyst beds are located in the distillation column,
Fig. 2 snows the process diagram of another process which alternative differs from that shown in Fig. 1 in that a part of the oligomerization product is withdrawn from a point above the column bottom and a part of the inert paraffins are withdrawn from a tray located in the upper section cf the column,
Fig. 3 shows the process diagram of a third process having a catalyst-filled prereactor placed preceding the distillation column shown in Fig. 1,
Fig. 4 shows the process diagram of a first embodiment of the invention having the catalyst beds placed in two side reactors so that the drawoff point from the distillation column to the first reactor is situated above the feed point of the olefin mixture,
Fig. 5 shows a second embodiment of the invention analogous to the alternative described above with the exception that the olefin mixture is fed to the second side reactor,
Fig. 6 shows the process diagram of a third embodiment of the invention having two side reactors arranged so that the feed of one reactor is heated in a heat exchanger by energy recovered from the product flow of the other reactor,
Fig. 7 shows the process diagram of a fourth embodiment cf the invention having the catalyst placed in both the distillation column and two side reactors,
Fig. 8 shows the process diagram of an fifth embodiment of the invention having the catalyst placed in a side reactor and having the recirculation between the distillation column and the side reactor implemented by virtue of thermosiphon action,
Fig. 9 shows the process diagram of a sixth embodiment of the invention having the catalyst placed in side reactor and having the recirculation between the distillation column and the side reactor implemented as forced circulation,
Fig. 10 shows the process diagram having two series connected distillation columns with multiple catalyst beds in both of them.

In the diagrams the reference numerals
1, 9, 18, 27, 41, 55, 70, 84, 95, 110 and 111 denote a column,
2, 10, 19, 28, 42, 56, 71, 85, 96 and 112 denote a feed point of the feedstock,
3, 11, 20, 29, 43, 57, 72, 86 and 97 denote a column tray,
4, 12, 21, 30, 44, 58, 73, 87, 98 and 113 denote a catalyst bed,
5, 13, 22, 33, 47, 61, 76, 89, 100, 114 and 119 denote a distillate condenser,
7, 15, 24, 35, 49, 63, 78, 91, 102, 116 and 121 denote a return point of the distillate,
6, 14, 23, 34, 48, 62, 77, 90, 101, 115 and 120 denote a boiler of the bottom product, and
8, 16, 25, 36, 50, 64, 79, 92, 103, 117 and 122 denote a return point of the vapor obtained from the boiler.

The meanings for the rest of the reference numerals will be evident from the following description.

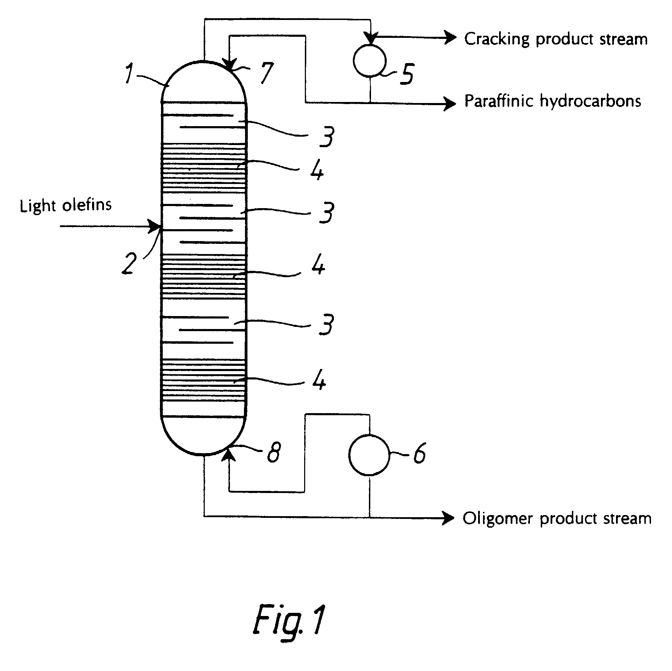

The general process principle is shown in Fig. 1. With reference to the diagram, an olefin-containing hydrocarbon mixture is fed directly to a distillation column 1 at an appropriately selected point (feed nozzle 2), possibly via a preheating stage. The column is provided with trays 3, e.g., valve trays between which catalyst layers 4 are adapted containing a zeolite catalyst. In the embodiment illustrated in the diagram, the feedstock is fed in the space remaining between the upper and middle layers of catalyst. In the column light olefins are oligomerized in a zeolite-catalyzed reaction forming primarily containing C₁₁ and heavier hydrocarbons, most of them unsaturated. Oligomerization is also accompanied by cracking reactions in which light hydrocarbons are formed.

The column top product flow contains light paraffinic hydrocarbons and possible light cracking products of the reaction. The column bottom product flow contains heavier hydrocarbons (oligomers) that are taken to further processing. These can be routed to, e.g., a separate hydrogenation process for improvement of product stability (when producing gasoline blending stock, diesel fuel or aliphatic solvents) or to distillation where the mixture of heavy olefins is separated into desired fractions according to intended uses. The maximum operating pressure of the column is determined by the desired composition of the bottom product (that is, diesel fuel, solvents, stock for lubrication oils). On the other hand, the maximum pressure of approx. 2500 kPa abs. in the column is dictated by the conditions required in distillation. At pressures above this, the lower section of the column has poor hydraulics, and fractionation in the column lower sectidn is almost nonexistent. The column upper section can be provided with a partial condenser 5 from which light hydrocarbons such as methane, ethane and ethylene as well as hydrogen formed in the cracking reactions are obtained in gaseous form. The column top provides a liquid flow primarily containing paraffins, since the olefins are consumed in oligomerization. When the partial condenser 5 is employed, also the paraffins can be obtained in gaseous form. The condenser is necessary only for attaining the reflux flow to the distillation column; in this case the liquid product flow will not be taken from the column top.

If the feedstock is comprised of a mixture of butenes and butanes, the partial condenser 5 at the column top provides the cracking products (methane, hydrogen, ethane, ethylene, propane and propene) in gaseous form, while the butanes are obtained as a liquid product. The butane stream obtained as the top product contains butenes to some extent. The amount of butenes in the top product depends on the degree of fractionation employed in the column and the placement of the catalyst. The higher the reflux ratio the more exhaustive is the consumption of butenes in the reactions. Depending on the operating temperature and pressure, the bottom product of the column is a product mixture primarily containing olefins but also paraffins formed in the side reactions. A portion of the energy consumed in distillation is introduced via a column bottom boiler 6, thus recycling the lighter hydrocarbons back to the column.

The olefinic hydrocarbon streams obtained from the catalytic distillation system are not as such directly suited for use as gasoline, diesel fuel or solvent. The octane number of the olefinic gasoline components is high, while due to their instability they can be hydrogenated into paraffinic components of a lower octane number. Also the instability of the diesel fuel and solvent fractions is removed in hydrogenation. As the hydrogenation inevitably causes changes in the carbon chain lengths to some extent, separation equipment such as distillation columns must be placed after the hydrogenation to obtain the desired product fractions. Hydrogenation can also be performed separately for each olefin fraction. In both cases the products are different kinds of paraffinic solvents as well as blending stock for diesel fuel, gasoline and possibly for lubricants. The proportions of said components in the final product are dependent on the oligomerization conditions.

In conjunction with all embodiments of the invention, it is possible to arrange drawoffs for both the oligomerized olefin streams and the paraffin-containing streams. The number of such streams can be one or greater. If the drawoff is located above the catalyst layers, a stream rich in light paraffins is obtained. Placement of the paraffins drawoff at a tray situated in the top section of the column facilitates purposeful design of the fractionation process of the cracking products and the paraffins. Correspondingly, a drawoff located below at least one catalyst layer provides a stream rich in oligomerized olefins, whereby at least two product fractions are obtained for hydrogenation: the drawoffs and the bottom product.

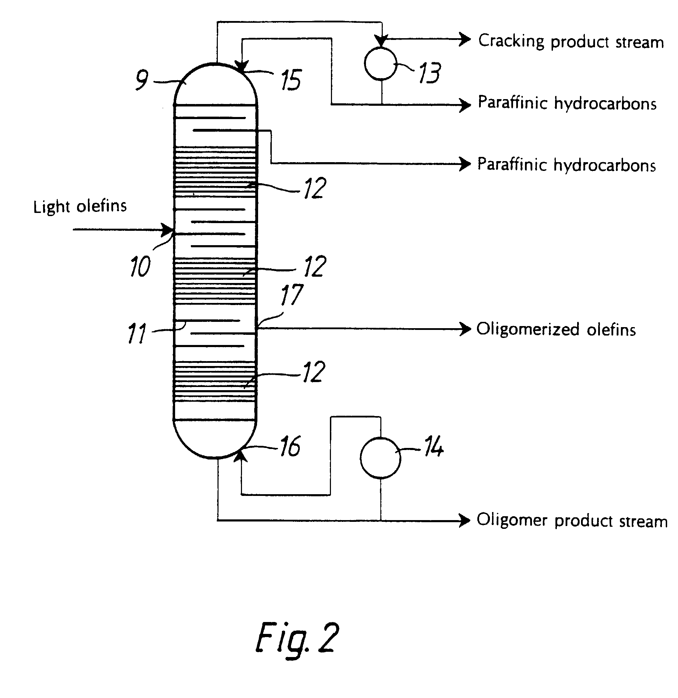

Fig. 2 elucidates the latter alternative arrangement. The column 9 with its trays 11 and reaction zones 12 is equivalent to that shown in Fig. 1, while also a distillate condenser 13 and a bottom boiler 14 are included. Reference numeral 17 denotes an oligomer flow drawoff, which is located above the lowermost reaction layer. This drawoff provides a fraction containing lighter oligomers than those of the bottom product.

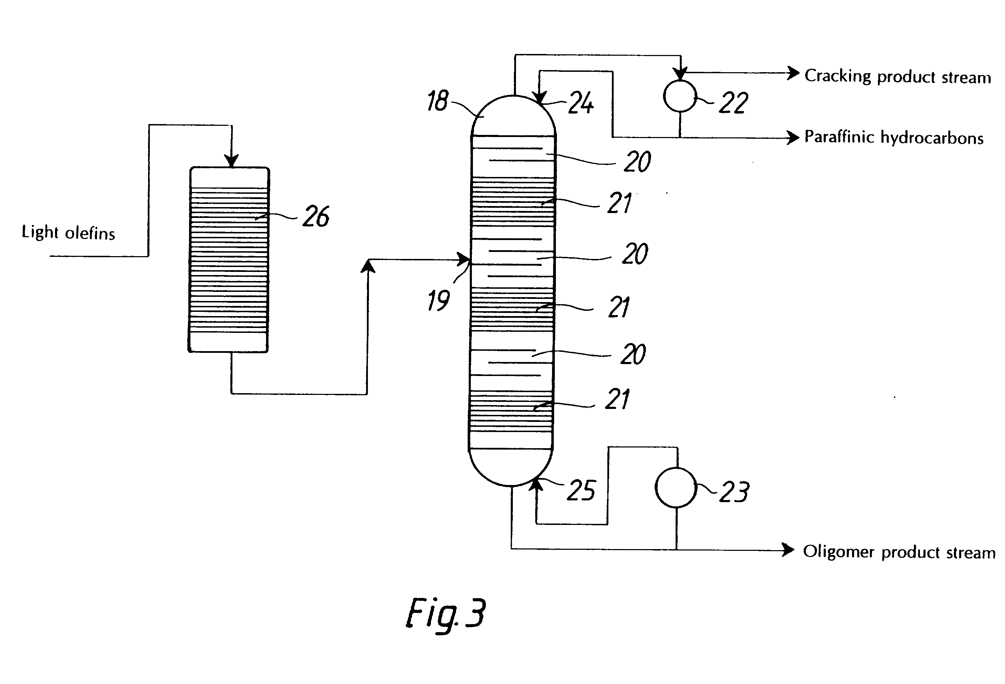

With reference to the embodiment of Fig. 3, the arrangement shown in Fig. 1 is complemented with a prereactor 26. The units 18 - 25 of this arrangement correspond to the units 1 - 7 of Fig. 1. The use of a prereactor is advantageous when the feed contains olefins and light paraffins (e.g. butenes and butanes). The prereactor 26 may comprise, e.g., a tubular reactor with a fixed catalyst bed. A benefit offered by the use of a prereactor is that, after a portion of the butenes has already become oligomerized, the boiling point of the mixture increases and the vapor pressure decreases, whereby the pressure of the column 30 need not be designed as high as for a case without the prereactor. The column temperature is determined by the pressure in the column. In order to support the desired reaction also in the column section of catalytic distillation, the column pressure must be such that keeps the column temperature within the operating region of the catalyst.

While the connection of the prereactor to the arrangement shown in Fig. 1 is illustrated in Fig. 3 alone, it must be understood that such a connection is possible with all other process embodiments described above or hereinafter.

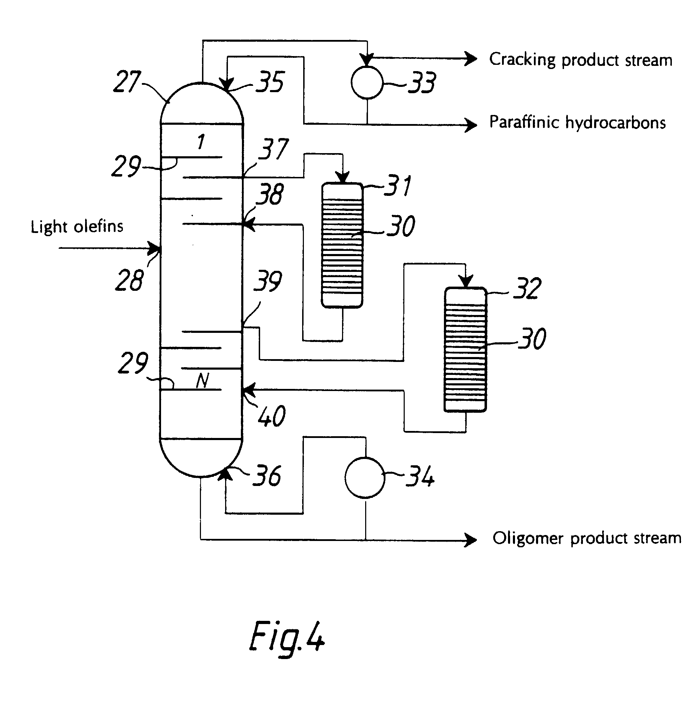

With reference to Fig. 1, the catalyst is placed directly in the distillation column itself. As the oligomerization reaction of this type can produce carbonaceous deposits on the catalyst, the embodiment shown in Fig. 4 has the catalyst 30 placed in reactors 31 and 32 externally from the column. Then, the distillation column can be provided with a desired number of side reactors of appropriate residence time. A benefit over the embodiment shown in Fig. 1 is that the catalyst contained in the reactor can be regenerated in a suitable order. For instance, three side reactors can be in operation concurrently, while the fourth is being regenerated.

With reference to the embodiment shown in Fig. 4, the olefins 28 are fed to a conventional distillation column 27 operated at a temperature of approx. 300 to 350 °C in the bottom (tray N) and at a temperature of, e.g., approx. 10 to 70 °C in the top (tray 1). The light olefins of the feedstock are vaporized in the column 27, rise upward and a portion of them is routed via a drawoff to a first side reactor 31, where they are contacted with the catalyst. The reactor is maintained at a temperature of, e.g., approx. 200 to 250 °C. The reaction product is removed from the reactor and returned to the column onto a tray below the initial drawoff tray. The column drawoff and the return point of the flow recycled from the side reactor are both located above the feed tray of the column feedstock. A side reactor connected in the above-described manner is hereinafter called the "upper side reactor". The column also has another side reactor 32 connected to it. Then, the flow to be routed to the latter reactor is taken from a drawoff located below the feed tray 28. Since the stream passing through the latter side reactor 32 is returned below the feed tray, the side reactor 32 can be called the "lower side reactor" analogously with the upper side reactor 31. For other parts of its system configuration, the second side reactor is fully equivalent to the first side reactor. The side reactors 31, 32 can be operated at a pressure above that of the column 28 (e.g., 50 bar vs. 5 bar). In the case shown in Fig. 4, the side reactors are fixed-bed reactors, whereby the liquid flow is arranged from top to bottom.

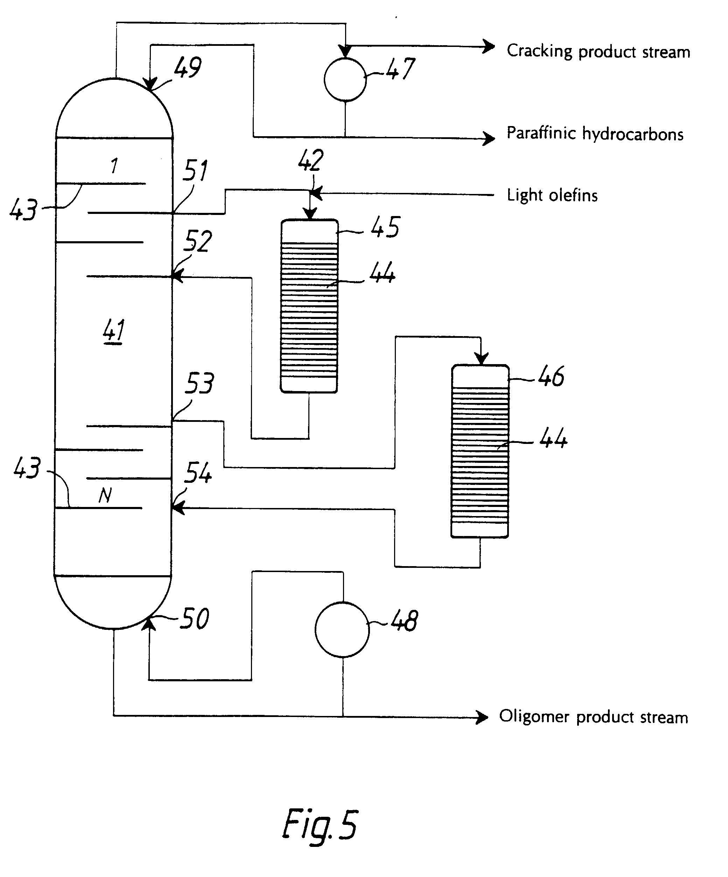

With reference to the embodiment of Fig. 5, the system arrangement is equivalent to that shown in Fig. 4 with the exception that the feed flow to the oligomerization process is routed partially - or as shown in Fig. 5 - entirely to side reactors 45, 46.

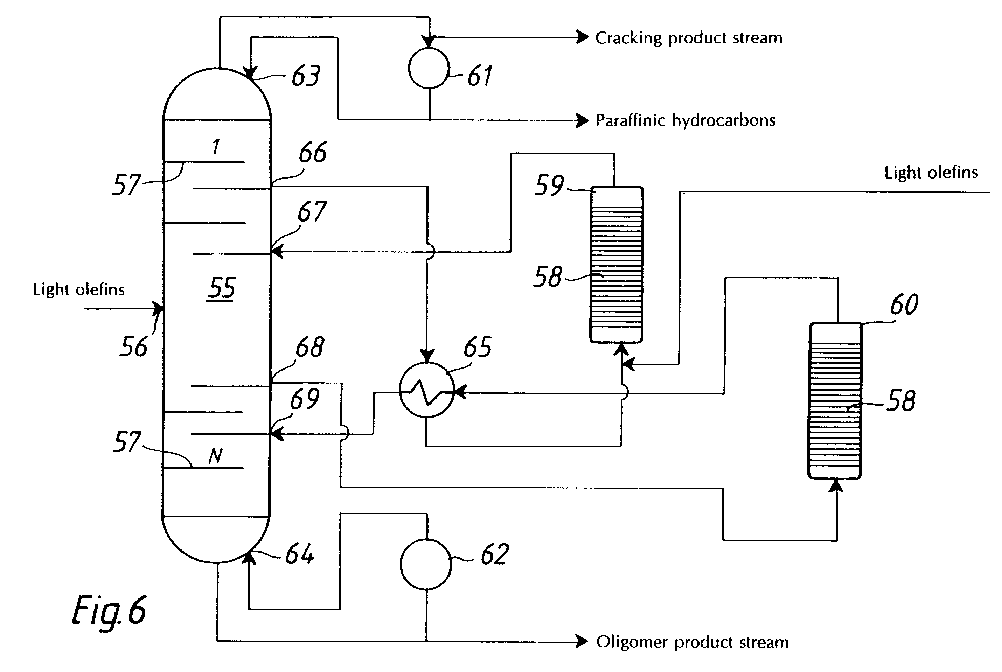

With reference to Fig. 6, the use of a heat exchanger is shown for transferring heat from the product flow of one side reactor to the feed of another side reactor. Then, the lower section of a column 55 is connected to a side reactor 60 (that is, a lower side reactor), whose product flow is taken via a heat exchanger 65 prior to returning the flow back to a distillation column 55. The colder side of the heat exchanger 65 is fed with a flow taken from a drawoff 66 and performing as feed for a side reactor 59 (upper side reactor) which is connected to the upper section of the column. By virtue of the heat exchanger 65 the feed flow can be heated to a desired temperature prior to routing it to the upper side reactor. This approach offers an optimal utilization of the reaction heats in a system based on the combination of a distillation column with side reactors. Instead of a separate heat exchanger 65, cooling pipes can be installed inside the catalyst layer of the side reactors 59, 60, the feed flow of one reactor being routed via said pipes into another reactor. Additionally, Fig. 6 shows an approach to feeding a portion of the feedstock to the first side reactor 59. Further shown in the diagram is the arrangement of routing the liquid flows which rise from the bottom upward through the side reactors. The reactors in the illustrated case can be tubular reactors, for instance.

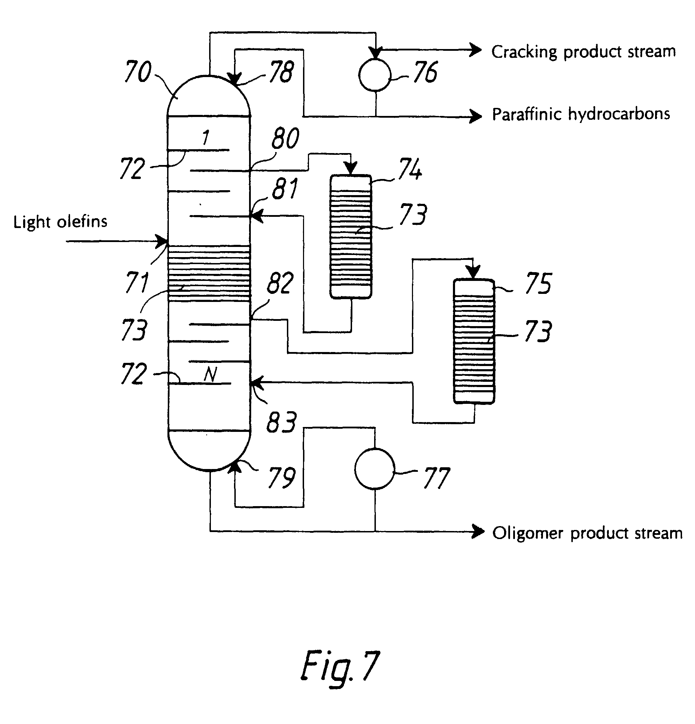

With reference to Fig. 7, a combination of the arrangements according to the diagrams of Figs. 1 and 4 is shown. In this system a part of the catalyst charge 73 is placed in the column 70, while the remaining part is placed in the side reactors 74, 75. The drawoff and return points 80, 81 of the first side reactor 74 are located above the reaction zone of the column, while the corresponding points of the second side reactor are situated below said reaction zone. The olefins are fed to the column above the reaction bed. With regard to the remaining details of the equipment arrangement, the system is identical to the configuration shown in Fig. 4, for instance. The above approach can provide drawoffs for sidestreams of differing compositions. However, the distillation column/reactor system has only one catalyst bed, whereby its regeneration requires shutdown of the column from production. The catalyst bed can be situated in the column either above the feed nozzle, or alternatively, multiple catalyst beds can be placed in the column.

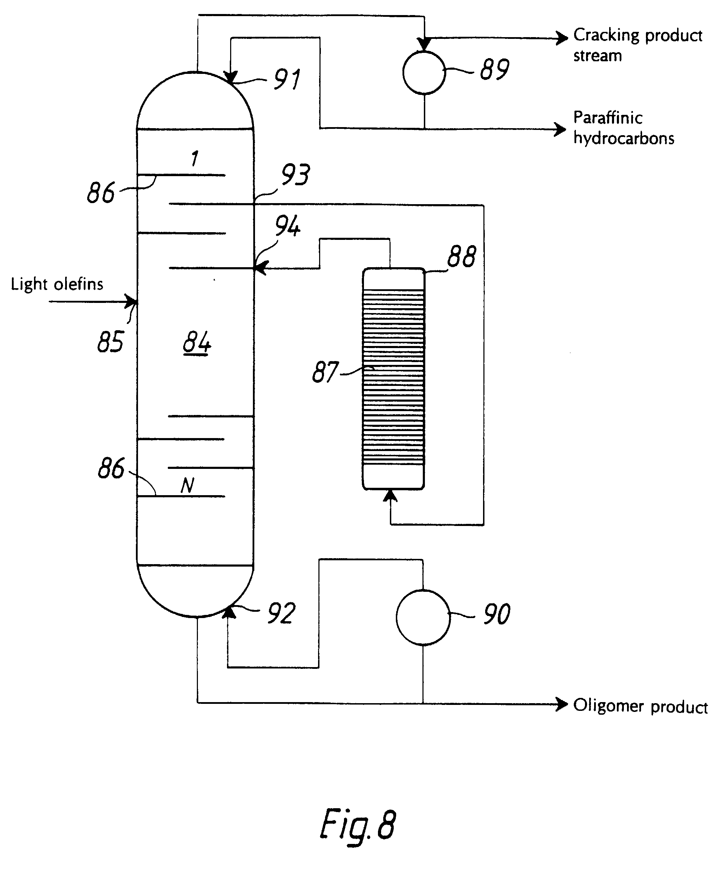

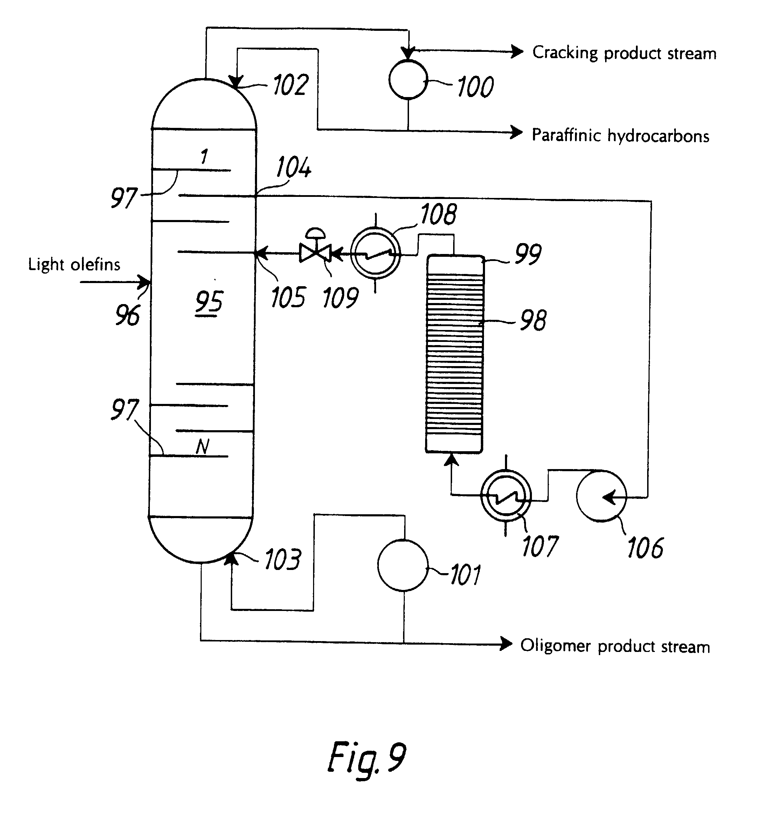

When using side reactors, the approach shown in Figs. 8 and 9, for instance, is possible. In the arrangement shown in Fig. 8 the exothermic reaction makes the side reactor function as a thermosiphon. Then, no pump is necessary for returning the outlet flow of the reactor back to the column. In the system shown in Fig. 9, a pump 106 is employed for pumping the reactor outlet flow back to the column. The need for a pump is dictated by the difference between the side reactor and the oulet and inlet flows of the column.

magnitude of the elevation difference between the side reactor inlet point of the flow from the column and the side reactor outlet point of the flow returning to the column. A further function of the pump is to compensate for the pressure drop in the side reactor catalyst bed. Changes in product composition are possible by way of altering the position of the connection points to the side reactors. Such alterations are reflected in the side reactor temperature and composition of the reaction product. Moreover, the use of side reactors makes it possible to control the space velocity, which affects the residence times in the reactors and thus, the product compositions.

In the side reactor arrangement according to Fig. 9, also heat exchangers 107 and 108 can be connected either before or after the reactor 99. The return flow from the reactor 99 back to the column 95 is taken via a back-pressure regulator 103. Such a combination of the pump 106, heat exchangers 107, 108 and back-pressure regulator 109 can be employed to run the reactor 99 under operating conditions different from those of the distillation column 95.

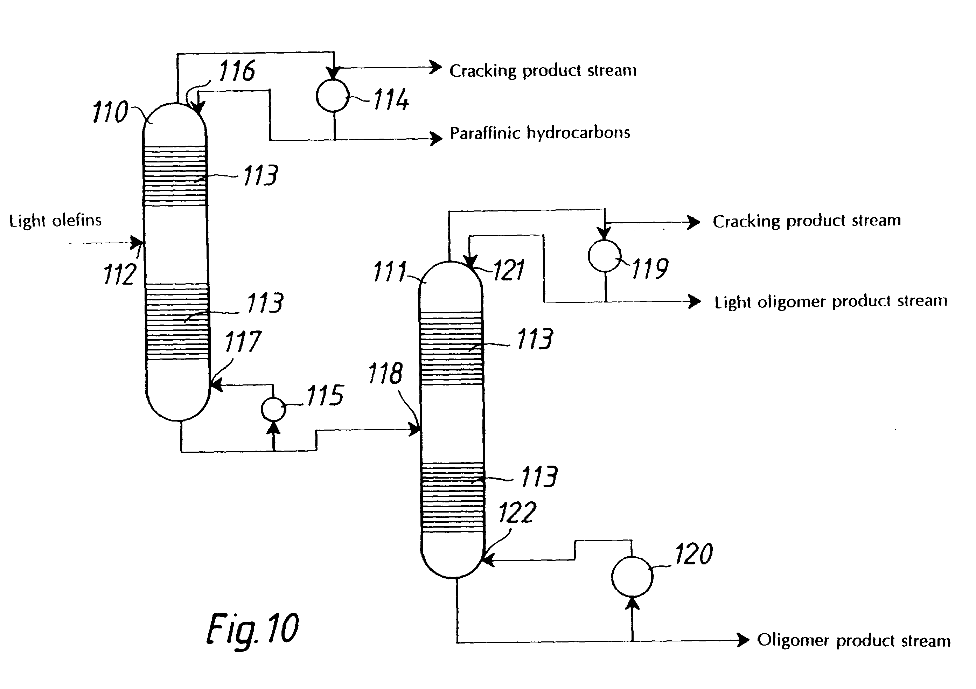

With reference to Fig. 10, an alternative embodiment is shown having the catalytic column divided in two. The first column 110 is operated at a higher pressure than the second column 111. The product composition can be altered by varying the column pressures. Such a two-column system can be complemented with a pre-reactors in accordance with Fig. 3, and the columns can be provided with drawoffs. The catalyst charge 113 can be placed either in the column(s), in the side reactors alone or in both the column(s) and in the side reactors.

If a heavier product is to be manufactured using any arrangement in accordance with the above-described embodiments, the bottom product of the column can be taken to a postreactor when needed.

During the optimization design of the column and the connected prereactors, side reactors and postreactors, catalysts of different operating temperatures can be desiredly placed to multiple locations in the system. Concomitantly, a disproportionate increase in column or reactor size is avoided.

Next, a few examples are given with test results.

### Example 1

### Feed of a mixture of C₄ olefins and C₄ paraffins directly to a catalytic distillation reactor system

A catalytic distillation reactor system according to Fig. 1 including three catalyst layers I, II and III was fed with a mixture of C₄ olefins and C₄ paraffins having a composition of

| | |
|---|---|
| i-butane | 26 % |
| n-butane | 14 % |
| 1-butene | 21 % |
| 2-butene | 39 % |

The catalyst was conventional ZSM-5 zeolite. The reaction beds were maintained at different temperatures so that the column lower section was at a temperature higher than that of the column upper section. The catalyst bed temperatures were as follows:

| | |
|---|---|
| Catalyst I | 265-280 °C |
| Catalyst II | 255-260 °C |
| Catalyst III | 180-220 °C |

The distillation reactor pressure was 20 bar.

The bottom product composition (as olefins) obtained after the oligomerization step was:
- C₄: = 0.29 %
- C₅₋₇: = 4.4 %
- C₈: = 3.9 %
- C₉₋₁₀: = 9.3 %
- C₁₁₊: = 77 %
- C₁₅₊: = 38 %

### Example 2

### Feed of C₄ olefins through a prereactor

The oligomerization reaction was performed using an arrangement in accordance with Fig. 3 by introducing a feed stream of pure 1-butene via a prereactor to a catalytic distillation reactor. A catalyst bed comprised of ZSM-5 zeolite was contained in both the prereactor and the catalytic distillation reactor. (The prereactor temperature was 240 °C and pressure 50 bar.

The product composition (olefins) after the prereactor was:
- C₄ =: 38 %
- C₅₋₇ =: 5.0 %
- C₈ =: 29 %
- C₉₋₁₀ =: 6.9 %
- C₁₁₊ =: 21 %

The product stream was next introduced to the catalytic distillation reactor, where it was oligomerized to obtain a product rich in lubricant blending stock fractions. The distillation reactor was in accordance with Example 1 provided with three reaction beds maintained at different temperatures so that the lower section of the distillation reactor was at a temperature higher than the reactor upper section. The temperatures in the reactor were as follows:

| | |
|---|---|
| Catalyst I | 265-280 °C |
| Catalyst II | 255-260 °C |
| Catalyst III | 180-220 °C |

The distillation reactor pressure was 7 bar.

Categorized by boiling point range, the product composition obtained from the distillation reactor was:
- TA - 80 °C: = 3 %
- 80 - 180 °C: = 25 %
- 180 - 200 °C: = 11 %
- 200 - 320 °C: = 47 %
- 320 °C - TL: = 14 %

As is evident from the above data, the catalytic distillation system according to the invention is well suited to the manufacture of heavy fractions. By reference it must be noted that a raffinate twice processed through a conventional tubular reactor contains less heavy fractions than the product obtained by virtue of the catalytic distillation system.

### Example 3

### Oligomerization of mixture of C₄ olefins and C₄ paraffins in a catalytic distillation system including side reactors

A mixture of C₄ olefins and paraffins was oligomerized using a distillation reactor system in accordance with Fig. 5 in which arrangement the fractions obtained from the distillation column are recycled via two catalytic side reactors back to the distillation column. The C₄ feed was introduced to the inlet of the upper side reactor in the manner shown in Fig. 5.

The feed composition was:

| | |
|---|---|
| i-butane | 26 % |
| n-butane | 14 % |
| 1-butene | 21 % |
| 2-butene | 39 % |

The side reactors had fixed catalyst beds in which the catalyst was ZSM-5 type zeolite.

The distillation reactor system was operated with a temperature differential between the parts of the system. Thus, the temperature at the distillation column bottom was 330 °C, while the column top temperature was 20 °C. The inlet flow temperature to the higher side reactor was 70 °C and to the lower side reactor 200 °C. Both catalytic side reactors were maintained at 230 °C. The distillation column pressure was approx. 5 bar, while the side reactors were run at 50 bar.

The product was fractionated by the following boiling point ranges:
- TA - 80 °C: = 4 %
- 80 - 180 °C: = 12 %
- 180 - 200 °C: = 6 %
- 200 - 320 °C: = 50 %
- 320 °C - TL: = 28 %

### Example 4

### Separation of lubricant blending stock fraction

The lubricant blending stock fraction was separated from the product obtained from Example 2 by distillation (at 410 °C and above) and said fraction was hydrogenated.

Analysis results:
Viscosity index 105
Pour point -36 °C

The thermal stability of lubricant blending stock fractions manufactured in the described manner was excellent. The thermal stability was even better than that of poly-alphaolefins and, contrary to these, the oligomer products manufactured according to the present invention were entirely free from catalyst residues.

### Example 5

### Oligomerization of a mixture of C₄ olefins, C₄ paraffines, propene and propane in a catalytic distillation system equipped with a side reactor, the reaction beds being provided with different catalysts

A mixture of C₄ olefins and paraffines (Raffinate II) together with propene was oligomerized in a distillation reaction system corresponding to Figure 5. Distillation fractions withdrawn from the distillation column were recirculated via two catalytic side reactors back to the column. The C₄ hydrocarbons as well as the C₃ hydrocarbons were fed into the feed flow of the upper side reactor as depicted in Figure 5. The feed of the lower side reactor only comprised a stream withdrawn from the distillation column.

The composition of the C₄ feed was the same as in Example 3. The C₃ feed contained about 80 % of propene, the rest being propane. The mass flow ratio of the C₃ feed to the C₄ feed was 23:103.

Fixed catalyst beds were used in the side reactors. The upper side reactor contained a ZSM-5 type zeolite as a catalyst, whereas the lower side reactor contained a HSiW (hetero polyacid) catalyst.

The different parts of the distillation reactor system were kept at different temperatures. Thus, the temperature at the bottom of the distillation column was 330 °C, whereas the temperature at the top was 20 °C. The temperature of the inlet flow to the upper side reactor was 70 °C and to the lower side reactor 200 °C. The upper side reactor, which contained the ZSM-5 catalyst, was maintained at 235 °C, whereas the lower side reactor, which contained the HSiW catalyst, was kept at 220 °C. The distillation column pressure was approx. 2.5 bar, while the side reactors were operated at 50 bar.

The product was fractionated by the following boiling point ranges:
- IBP - 200 °C: = 31 %
- 200 - 350 °C: = 60 %
- 350 - 410 °C: = 6 %
- 410 - FBP °C: = 3 %

The cetane number of the distillate fraction of 200 to 350 °C was 50. Both the 350 to 410 °C and the 410+ °C fractions were, subject to hydrogenation, suitable for use as lubricants.

This Example clearly shows that different oligomerization catalyst can be used in the reactors of the process. Furthermore, the Example indicates that the system according to the invention can be run on a mixture of propene and butenes as feedstock.

### Example 6

### Oligomerization of propene in a catalytic distillation reactor system

Propene containing about 20 % of propane as an impurity was oligomerized in a distillation reactor system depicted in Figure 5. Distillation fractions withdrawn from the distillation column were recirculated via two catalytic side reactors back to the column. The propene was fed into the feed flows of both side reactors, as shown for the upper side reactor in Figure 5. The propene feed of the upper side reactor was twice the amount of the propene feed of the lower side reactor.

The side reactors contained fixed reaction beds, whose catalyst comprised a ZSM-5 type catalyst.

The conditions of the distillation reaction system were approximately the same as in Example 5, with the exception for the temperatures of the side reactors: both reactors were maintained at a temperature in the range from 220 to 230 °C.

The product was fractionated by the following boiling point ranges:
- IBP - 200 °C: = 15 %
- 200 - 350 °C: = 72 %
- 350 - 410 °C: = 10 %
- 410 - FBP °C: = 3 %

The lubricant 410+ °C fraction was hydrogenated and its viscosity index was determined. It turned out that the described reactor system produced a lubricant fraction having a viscosity index of 123, i.e. a better viscosity index than that obtained in Example 4. The reason for this improvement would appear to be that an oligomer product produced from propene is branched in a different way than an oligomer product produced from butenes.

The pour point of the lubricant fraction was -54 °C.

## Claims

1. A process for oligomerization of olefins, according to which process C₃ to C₂₀ olefins contained in the feedstock are subjected to an oligomerization reaction in the presence of a catalyst in order to produce a product containing oligomers,
**characterized** in that
- the oligomerization reaction is carried out in a catalytic distillation system comprising a distillation column (27; 41; 55; 70; 84; 95) for product fractionation, and connected thereto, at least one side reactor (31, 32; 45, 46; 59, 60; 74, 75; 88; 99), in which an essential part of the catalyst of the catalytic distillation system is placed, said catalyst comprising a solid oligomerization catalyst which forms a catalyst layer (30; 44; 58; 69; 73; 87; 98),
- at least 20 % of the internal liquid flow of the distillation column is routed through said catalyst layer (30; 44; 58; 69; 73; 87; 98), and
- the portion of internal liquid flow of the column routed through said catalyst layer is recycled back to the distillation column (27; 41; 55; 70; 84; 95).

2. The process according to claim 1, wherein there are 1 to 20, preferably about 2 to 10 side reactors (31, 32; 45, 46; 59, 60; 74, 75; 88; 99).

3. The process according to claim 1, wherein 40 to 90 % of the internal liquid flow of the column (27; 41; 55; 70; 84; 95) is routed through said catalyst layer (30; 44; 58; 69; 73; 87; 98).

4. The process according to claim 1, wherein the catalyst used is of the ZSM-5 type.

5. The process according to claim 4, wherein the oligomerization reaction is performed at a temperature in the range from 150 to 350 °C.

6. The process according to claim 1, wherein the recycled portion of the internal liquid flow is returned to the distillation column (27; 41; 55; 70; 84; 95) to a point advantageous for the distillation step.

7. The process according to claim 6, wherein, having passed through said catalyst layer, said liquid flow is recycled back to said column to a return tray below the drawoff tray of said liquid flow, whereby the location of said return tray is chosen so as to attain desired fractionation of the components contained in the recycled flow.

8. The process according to claim 6, wherein the liquid flow withdrawn from a drawoff point (37; 66; 80) above the column feed tray (28; 56; 71) is routed to a side reactor (31; 59; 74), and having passed through the catalyst layer, it is recycled back to the column to a point above said feed tray, while yet below said drawoff point (38; 67; 81) of said liquid flow.

9. The process according to claim 6, wherein the liquid flow withdrawn from a drawoff point (39; 68; 82) below the feed tray (28; 56; 71) of the column is routed to a side reactor (32; 60; 75), and having passed through the catalyst layer, it is recycled back to the column to a point below said drawoff point (40; 69; 83) of said liquid flow.

10. The process according to claim 1, based on the use of at least two side reactors, wherein the sidestream to the first reactor (31; 59; 74) is withdrawn from above the feed tray of the reactants, while the sidestream to a second reactor (32; 60; 75) is withdrawn from below said feed tray of the reactants.

11. The process according to claim 10, wherein the product stream of said first reactor (31; 59; 74) is recycled back to the column to a point above the feed tray (28; 56; 71) of reactants, while the product stream of said second reactor (32; 60; 75) is recycled back to the column to a point below said feed tray of reactants.

12. The process according to any one of the previous claims, wherein the sidestream withdrawn from said column (95) is recycled by thermosiphon action via said reactor (99) back to said distillation column.

13. The process according to claim 1, wherein at least a portion of the reactant feedstock is fed directly to at least one side reactor (45).

14. The process according to claim 13, wherein a substantial portion of the reactant feedstock is fed to such a side reactor (45) which during the startup of the system or change of product grade is entirely disconnected from receiving a sidestream from the distillation column (41).

15. The process according to 1, wherein at least one of the reactors (31, 32; 45, 46; 59, 60; 74, 75; 88; 99) is operated at temperature and/or pressure conditions different from those of the distillation column (27; 41; 55; 70; 84; 95).

16. The process according to claim 15, wherein the pressure of at least one of said reactors (31, 32; 45, 46; 59, 60; 74, 75; 88; 99) is maintained at 10 to 100 bar, while the pressure of said distillation column (27; 41; 55; 70; 84; 95) is maintained at 1 to 10 bar.

17. The process according to claim 1, based on the use of at least two side reactors, wherein at least a portion of the heat contained in the outlet flow of one side reactor (60) is transferred to the feed flow of the other side reactor (59).

18. The process according to claim 1, wherein the oligomer-containing product is withdrawn from the distillation column/reactor system (9, 12) from a tray (17) situated below the feed tray (10), said withdrawal tray being selected so as to render a desired product composition.

19. The process according to any one of the previous claims, wherein the product obtained from oligomerization reaction is routed to a postreactor (111) to the end of obtaining conversion into a yet heavier product.

20. The process according to claim 1, wherein the distillation column (70) includes at least one catalyst layer (73).

21. The process according to claim 1, which comprises using a catalytic distillation system which includes at least two catalyst layers (30; 44; 58; 69; 73), wherein at least one of the catalyst layers contains a different type of a catalyst than the other layers.

22. The process according to any one of the previous claims, wherein the side reactor comprises a flow reactor, in which the reactants and the reaction products flow in the same direction.

23. The process according to any one of the previous claims, wherein all the reaction products are withdrawn from the side reactor from one point.

## Patentansprüche

1. Verfahren zur Oligomerisation von Olefinen, gemäß dem die im Ausgangsmaterial enthaltenen C₃-C₂₀-Olefine zwecks Herstellung eines oligomerhaltigen Produktes einer Oligomerisationsreaktion in Gegenwart eines Katalysators unterworfen werden, dadurch **gekennzeichnet**, daß
- die Oligomerisationsreaktion in einem katalytischen Destillationssystem durchgeführt wird, umfassend eine Destillationskolonne (27; 41; 55; 70; 84; 95) zur Produktfraktionierung und mindestens einen daran angeschlossenen Seitenreaktor (31, 32; 45, 46; 59, 60; 74, 75; 88; 99), in welchem ein wesentlicher Teil des Katalysators des katalytischen Destillationssystems untergebracht ist, welcher Katalysator einen festen Oligomerisationskatalysator umfaßt, der eine Katalysatorschicht (30; 44; 58; 69; 73; 87; 98) bildet,
- mindestens 20 % des internen Flüssigkeitsstromes in der Destillationskolonne durch die Katalysatorschicht (30; 44; 58; 69; 73; 87; 98) geführt wird, und
- der durch diese Katalysatorschicht geführte Anteil des internen Flüssigkeitsstromes in der Kolonne zur Destillationskolonne (27; 41; 55; 70; 84; 95) rückgeführt wird.

2. Verfahren nach Anspruch 1, wobei 1 bis 20, vorzugsweise 2 bis 10 Seitenreaktoren (31, 32; 45, 46; 59, 60; 74, 75; 88; 99) eingesetzt werden.

3. Verfahren nach Anspruch 1, wobei 40 bis 90 % des internen Flüssigkeitsstromes in der Kolonne (27; 41; 55; 70; 84; 95) durch die Katalysatorschicht (30; 44; 58; 69; 73; 87; 98) geführt wird.

4. Verfahren nach Anspruch 1, wobei der eingesetzte Katalysator vom Typ ZSM-5 ist.

5. Verfahren nach Anspruch 4, wobei die Oligomerisationsreaktion bei einer Temperatur im Bereich von 150 bis 350 °C durchgeführt wird.

6. Verfahren nach Anspruch 1, wobei der rückgeführte Anteil des internen Flüssigkeitsstromes zur Destillationskolonne (27; 41; 55; 70; 84; 95) an einen für die Destillation geeigneten Punkt zurückgeführt wird.

7. Verfahren nach Anspruch 6, wobei der Flüssigkeitsstrom nach seinem Lauf durch die Katalysatorschicht zur Kolonne an einen Rückführboden unterhalb des Abzugsbodens des Flüssigkeitsstromes rückgeführt wird, wobei die Lage des Rückführbodens so gewählt wird, daß die gewünschte Fraktionierung der in dem Rückführstrom enthaltenen Komponenten erreicht wird.

8. Verfahren nach Anspruch 6, wobei der von einem Abzugspunkt (37; 66; 80) über dem Kolonnenbeschickungsboden (28; 56; 71) abgezogene Flüssigkeitsstrom zu einem Seitenreaktor (31; 59; 74) geführt wird und nach seinem Lauf durch die Katalysatorschicht zur Kolonne an einen Punkt über dem Beschickungsboden, jedoch unterhalb des Abzugspunktes (38; 67; 81) des Flüssigkeitsstromes, rückgeführt wird.

9. Verfahren nach Anspruch 6, wobei der von einem Abzugspunkt (39; 68; 82) unter dem Kolonnenbeschickungsboden (28; 56; 71) abgezogene Flüssigkeitsstrom zu einem Seitenreaktor (32; 60; 75) geführt wird und nach seinem Lauf durch die Katalysatorschicht zur Kolonne an einen Punkt unterhalb des Abzugspunktes (40; 69; 83) des Flüssigkeitsstroms rückgeführt wird.

10. Verfahren nach Anspruch 1, basierend auf dem Einsatz mindestens zweier Seitenreaktoren, wobei der Seitenstrom zum ersten Reaktor (31; 59; 74) von oberhalb des Beschikkungsbodens der Reaktanten abgezogen wird, während der Seitenstrom eines zweiten Reaktors (32; 60; 75) von unterhalb dieses Beschickungsbodens der Reaktanten abgezogen wird.

11. Verfahren nach Anspruch 10, wobei der Produktstrom des ersten Reaktors (31; 59; 74) zur Kolonne an einen Punkt oberhalb des Beschickungsbodens (28; 56; 71) der Reaktanten rückgeführt wird, während der Produktstrom des zweiten Reaktors (32; 60; 75) zur Kolonne an einen Punkt unterhalb des Beschickungsbodens der Reaktanten rückgeführt wird.

12. Verfahren nach einem der vorstehenden Ansprüche, wobei der von der Kolonne (95) abgezogene Seitenstrom durch Thermosiphonwirkung über den Reaktor (99) zurück zur Destillationskolonne geführt wird.

13. Verfahren nach Anspruch 1, wobei zumindest ein Teil des Reaktantenausgangsmaterials direkt mindestens einem Seitenreaktor (45) zugeführt wird.

14. Verfahren nach Anspruch 13, wobei ein wesentlicher Teil des Reaktantenausgangsmaterials einem derartigen Seitenreaktor (45) zugeführt wird, der während des Startens des Systems oder des Änderns der Produktklasse völlig von dem Empfang eines Seitenstromes von der Destillationskolonne (41) ausgeschlossen ist.

15. Verfahren nach Anspruch 1, wobei mindestens einer der Reaktoren (31, 32; 45, 46; 59, 60; 74, 75; 88; 99) unter Temperatur- und/oder Druckverhältnissen betrieben wird, die sich von denen in der Destillationskolonne (27; 41; 55; 70; 84; 95) unterscheiden.

16. Verfahren nach Anspruch 15, wobei der Druck mindestens eines Reaktors (31, 32; 45, 46; 59, 60; 74, 75; 88; 99) bei 10 bis 100 bar gehalten wird, während der Druck der Destillationskolonne (27; 41; 55; 70; 84, 95) bei 1 bis 10 bar gehalten wird.

17. Verfahren nach Anspruch 1, basierend auf dem Einsatz mindestens zweier Seitenreaktoren, wobei mindestens ein Teil der im Abzugsstrom eines Seitenreaktors (60) enthaltenen Wärme in den Beschickungsstrom des anderen Seitenreaktors (59) übertragen wird.

18. Verfahren nach Anspruch 1, wobei das oligomerhaltige Produkt von der Destillationskolonne/dem Reaktorsystem (9, 12) von einem Boden (17) unterhalb des Beschickungsbodens (10) abgezogen wird, wobei der Abzugsboden so gewählt wird, daß eine gewünschte Produktzusammensetzung erhalten wird.

19. Verfahren nach einem der vorstehenden Ansprüche, wobei das aus der Oligomerisationsreaktion erhaltene Produkt zwecks Umwandlung in ein noch schwereres Produkt zu einem Nachreaktor (111) geführt wird.

20. Verfahren nach Anspruch 1, wobei die Destillationskolonne (70) mindestens eine Katalysatorschicht (73) enthält.

21. Verfahren nach Anspruch 1, umfassend den Einsatz eines katalytischen Destillationssystems einschließlich mindestens zweier Katalysatorschichten (30; 44; 58; 69; 73), wobei mindestens eine der Katalysatorschichten einen Katalysator anderen Typs enthält als die anderen Schichten.

22. Verfahren nach einem der vorstehenden Ansprüche, wobei der Seitenreaktor einen Fließreaktor umfaßt, in welchem die Reaktanten und die Reaktionsprodukte in dieselbe Richtung fließen.

23. Verfahren nach einem der vorstehenden Ansprüche, wobei sämtliche Reaktionsprodukte von einem Punkt aus vom Seitenreaktor abgezogen werden.

## Revendications

1. Procédé pour l'oligomérisation d'oléfines, procédé selon lequel les oléfines en C₃ à C₂₀ contenues dans un produit de départ sont soumises à une réaction d'oligomérisation en présence d'un catalyseur de façon à donner un produit contenant des oligomères, caractérisé en ce que
- la réaction d'oligomérisation est effectuée dans un système de distillation catalytique comprenant une colonne de distillation (27, 41, 55, 70, 84, 95) pour le fractionnement des produits, et reliée à elle, au moins un réacteur latéral (31, 32, 45, 46, 59, 60, 74, 75, 88, 99) dans lequel est placée la partie essentielle du catalyseur du système de distillation catalytique, ledit catalyseur comprenant un catalyseur solide d'oligomérisation qui forme une couche catalytique (30, 44, 58, 69, 73, 87, 98),
- au moins 20 % du flux liquide interne de la colonne de distillation est passé à travers ladite couche de catalyseur (30, 44, 58, 69, 73, 87, 98) et
- la portion de flux liquide interne de la colonne passée à travers ladite couche de catalyseur est renvoyée en recyclage vers la colonne de distillation (27, 41, 55, 70, 84, 95).

2. Procédé selon la revendication 1, dans lequel il y a 1 à 20, de préférence environ 2 à 10 réacteurs latéraux (31, 32, 45, 46, 59, 60, 74, 75, 88, 99).

3. Procédé selon la revendication 1, dans lequel 40 à 90 % du flux liquide interne de la colonne (27, 41, 55, 70, 84, 95) est passé à travers ladite couche de catalyseur (30, 44, 58, 69, 73, 87, 98).

4. Procédé selon la revendication 1, dans lequel le catalyseur utilisé est du type ZSM-5.

5. Procédé selon la revendication 4, dans lequel la réaction d'oligomérisation est effectuée à une température comprise entre 150°C et 350°C.

6. Procédé selon la revendication 1, dans lequel la portion recyclée du flux liquide interne est retournée dans la colonne de distillation (27, 41, 55, 70, 84, 95) en un point avantageux pour l'étape de distillation.

7. Procédé selon la revendication 6, dans lequel, après être passé à travers la couche de catalyseur, ledit flux liquide est retourné en recyclage dans ladite colonne sur un plateau de retour au dessous du plateau de soutirage dudit flux liquide, grâce à quoi l'emplacement dudit plateau de retour est choisi de façon à obtenir le fractionnement des composants contenus dans le flux recyclé.

8. Procédé selon la revendication 6, dans lequel le flux liquide soutiré à partir d'un point de soutirage (37, 66, 80) au dessus du plateau d'alimentation de la colonne (28, 56, 71) est passé dans un réacteur latéral (31,59, 74) et après être passé à travers la couche de catalyseur, il est retourné en recyclage dans la colonne en un point situé au dessus dudit plateau d'alimentation tout en étant au dessous dudit point de soutirage (38, 67, 81) dudit flux liquide.

9. Procédé selon la revendication 6, dans lequel le flux liquide soutiré à partir d'un point de soutirage (39, 68, 82) au dessous du plateau d'alimentation (28, 56, 71) de la colonne est passé dans un réacteur latéral (32, 60, 75) et après avoir traversé la couche de catalyseur, il est retourné en recyclage dans la colonne en un point situé au dessous du point de soutirage (40, 69, 86) dudit flux liquide.

10. Procédé selon la revendication 1, basé sur l'utilisation d'au moins deux réacteurs latéraux dans lequel le courant latéral vers le premier réacteur (31, 59, 74) est soutiré depuis un point situé au dessus du plateau d'alimentation des réactants tandis que le courant latéral vers un second réacteur (32, 60, 75) est soutiré depuis un point situé au dessous du plateau d'alimentation des réactants.

11. Procédé selon la revendication 10, dans lequel le courant de produits dudit premier réacteur (31, 59, 74) est renvoyé en recyclage dans la colonne en un point situé au dessus du plateau d'alimentation (28, 56, 71) des réactants tandis que le courant de produits dudit second réacteur (32, 60, 75) est renvoyé en recyclage dans la colonne en un point situé au dessous dudit plateau d'alimentation des réactants.

12. Procédé selon l'une quelconque des revendications précédentes, dans lequel le courant latéral soutiré depuis ladite colonne (95) est recyclé par effet de thermosiphon via ledit réacteur (99) en retour vers ladite colonne de distillation.

13. Procédé selon la revendication 1, dans lequel au moins une portion de l'alimentation en réactants est introduite directement dans au moins un réacteur latéral (45).

14. Procédé selon la revendication 13, dans lequel une portion substantielle de l'alimentation en réactants est introduite dans un tel réacteur latéral (45) qui, au cours du démarrage du système ou au cours du changement de qualité de produit est complètement coupé de la réception d'un courant latéral provenant de la colonne de distillation (41).

15. Procédé selon la revendication 1, dans lequel au moins un des réacteurs (31, 32, 45, 46, 59, 60, 74, 75, 88, 99) est conduit dans des conditions de température et/ou de pression différentes de celles de la colonne de distillation (27, 41, 55, 70, 84, 95).

16. Procédé selon la revendication 15, dans lequel la pression d'au moins un desdits réacteurs (31, 32, 45, 46, 59, 60, 74, 75, 88, 99) est maintenue entre 10 et 100 bars tandis que la pression de ladite colonne de distillation (27, 41, 55, 70, 84, 95) est maintenue entre 1 et 10 bars.

17. Procédé selon la revendication 1, basé sur l'utilisation d'au moins deux réacteurs latéraux, dans lequel au moins une portion de la chaleur contenue dans le flux de sortie d'un des réacteurs latéraux (60) est transférée vers le flux d'alimentation de l'autre réacteur latéral (59).

18. Procédé selon la revendication 1, dans lequel le produit contenant les oligomères est soutiré depuis le système colonne de distillation/réacteur (9,12) à partir d'un plateau (17) situé au dessous du plateau d'alimentation (10), ledit plateau de soutirage étant choisi de façon à donner une composition de produit souhaitée.

19. Procédé selon l'une quelconque des revendications précédentes, dans lequel le produit obtenu à partir de la réaction d'oligomérisation est passé dans un post-réacteur (111) afin d'obtenir la conversion en un produit encore plus lourd.

20. Procédé selon la revendication 1, dans lequel la colonne de distillation (70) comprend au moins une couche de catalyseur (73).

21. Procédé selon la revendication 1, comprenant l'utilisation d'un système de distillation catalytique qui inclut au moins deux couches de catalyseur (30, 44, 58, 69, 73) parmi lesquelles au moins une desdites couches de catalyseur contient un type de catalyseur différent de celui des autres couches.

22. Procédé selon l'une quelconque des revendications précédentes, dans lequel le réacteur latéral comprend un réacteur à flux dans lequel les réactants et le flux des produits réactionnels coulent dans la même direction.

23. Procédé selon l'une quelconque des revendications précédentes, dans lequel tous les produits de la réaction sont soutirés du réacteur latéral à partir d'un point.
